# EUROPEAN PATENT SPECIFICATION

(11) **EP 2 675 354 B1**
(45) Date of publication and mention of the grant of the patent: **07.01.2015**
(21) Application number: 12706694.2
(22) Date of filing: 14.02.2012
(51) Int. Cl.: A61B 5/06, A61B 5/0402

(54) **SYSTEM FOR PROVIDING AN ELECTRICAL ACTIVITY MAP USING OPTICAL SHAPE SENSING**
SYSTEM ZUR BEREITSTELLUNG EINER KARTE ELEKTRISCHER AKTIVITÄTEN DURCH OPTISCHE FORMMESSUNG
SYSTÈME PERMETTANT D'OBTENIR UNE CARTE D'ACTIVITÉ ÉLECTRIQUE À L'AIDE D'UNE DÉTECTION DE FORME OPTIQUE

(30) Priority: 17.02.2011 EP 11154871
(43) Date of publication of application: 25.12.2013
(73) Proprietor: Koninklijke Philips N.V., 5656 AE Eindhoven (NL)
(72) Inventor: GIJSBERS, Gerardus, Henricus, Maria, NL-5656 AE Eindhoven (NL); VAN DEN BRINK, Hendrikus, Bernardus, NL-5656 AE Eindhoven (NL); SLEGT, Sander, NL-5656 AE Eindhoven (NL); NIJHOF, Niels, NL-5656 AE Eindhoven (NL); DIJKKAMP, Dirk, NL-5656 AE Eindhoven (NL)
(74) Representative: Steffen, Thomas
(86) International application number: PCT/IB2012/050653
(87) International publication number: WO 2012/110942

(56) References cited:
- WO-A1-2009/023801
- WO-A1-2011/098926
- WO-A1-2011/141830
- CHENG L K ET AL: "RAPID CONSTRUCTION OF A PATIENT-SPECIFIC TORSO MODEL FROM 3D ULTRASOUND FOR NON-INVASIVE IMAGING OF CARDIAC ELECTROPHYSIOLOGY", MEDICAL AND BIOLOGICAL ENGINEERING AND COMPUTING, vol. 43, no. 3, 1 May 2005 (2005-05-01), pages 325-330, XP001508007, ISSN: 0140-0118, DOI: 10.1007/BF02345808
- CHENG ET AL: "Construction of Patient Specific Geometries Suitable for the Inverse Problem of Electrocardiography", PROCEEDINGS OF THE 2005 IEEE ENGINEERING IN MEDICINE AND BIOLOGY 27T H ANNUAL INTERNATIONAL CONFERENCE, SHANGHAI, CHINA, 01-04 SEPT. 2005, 1 January 2005 (2005-01-01), pages 7201-7203, XP031000877, DOI: 10.1109/IEMBS.2005.1616170 ISBN: 978-0-7803-8741-6
- MICHAEL E CAIN ET AL: "Detection of the Fingerprint of the Electrophysiological Abnormalities that Increase Vulnerability to Life-Threatening Ventricular Arrhythmias", JOURNAL OF INTERVENTIONAL CARDIAC ELECTROPHYSIOLOGY, vol. 9, no. 2, 1 October 2003 (2003-10-01) , pages 103-118, XP019208687, ISSN: 1572-8595, DOI: 10.1023/A:1026259702892
- GHANEM R N ET AL: "Heart-surface reconstruction and ECG electrodes localization using fluoroscopy, epipolar geometry and stereovision: Application to noninvasive imaging of cardiac electrical activity", IEEE TRANSACTIONS ON MEDICAL IMAGING, vol. 22, no. 10, 1 October 2003 (2003-10-01), pages 1307-1318, XP011101829, ISSN: 0278-0062, DOI: 10.1109/TMI.2003.818263

## Description

### FIELD OF THE INVENTION

The invention relates to a system, a method and a computer program for providing an electrical activity map of the heart of a living being by means of electrical signals from the heart acquired by a plurality of surface electrodes being arranged on an outer surface of the living being. The invention relates further to a vest comprising the plurality of surface electrodes.

### BACKGROUND OF THE INVENTION

The article "Noninvasive Characterization of Epicardial Activation in Humans With Diverse Atrial Fibrillation Patterns" by P.S. Cuculich et al., Circulation, Journal of the American Heart Association, 122, pages 1364 to 1372 (2010) discloses a system comprising an electrode vest with surface electrodes for measuring electrical potentials on an outer surface of a person. The system further comprises a reconstruction unit for reconstructing epicardial electrical potentials based on i) a spatial relation between the heart surface and the surface electrodes on the outer surface of the person and ii) the measured electrical potentials. The spatial relation between the heart surface and the surface electrodes on the outer surface of the person are obtained by acquiring an x-ray computed tomography image showing both, the heart surface and the surface electrodes on the outer surface of the person. By acquiring the x-ray computed tomography image a relatively high radiation dose is applied to the person.

The article by L. K. Cheng et al., "Rapid construction of a patient-specific torso model from 3D ultrasound for non-invasive imaging of cardiac electrophysiology", Med. Biol. Eng. Comput., 2005, vol. 43, p. 325-330, discloses a system for providing an electrical activity map using 256 surface electrodes embedded in a vest. The positions of the plurality of surface electrodes are determined by means of a hand-held laser scanner, the orientation and position of which is determined by an embedded magnetic tracking system. The system further comprises a cardiac structure position determination unit and an electrical activity map determination unit. An ultrasound probe is used to determine the cardiac structure position, wherein the location and orientation of the ultrasound probe are reconstructed using a magnetic tracking device.

WO 2009/023801 A1 discloses that one or more optical shape sensing fibers may be coupled to an elongate medical instrument in order to determine its orientation and position, and that optical shape sensing fibers may also advantageously be attached to a patient patch in order to determine patient movement e.g. caused by respiration.

### SUMMARY OF THE INVENTION

It is an object of the present invention to provide a system, a method and a computer program for providing an electrical activity map of the heart of a living being by means of electrical signals from the heart acquired by a plurality of surface electrodes being arranged on an outer surface of the living being, wherein the radiation dose applied to the person can be reduced, in particular, can be eliminated. It is a further object of the present invention to provide a vest for being worn by a living being and for being used for providing the electrical activity map.

In a first aspect of the present invention a vest for being worn by a living being is presented, the vest being adapted to be used for providing an electrical activity map, the vest comprising:
- a plurality of surface electrodes for being arranged on an outer surface of the living being, when the vest is worn by the living being, and for acquiring electrical signals from the heart of the living being,
- an optical shape sensing fiber for generating an optical shape sensing signal being indicative of the three-dimensional shape of the optical shape sensing fiber and for providing the optical shape sensing signal to a surface electrodes positions calculation unit.

In a further aspect of the present invention a system for providing an electrical activity map of the heart of a living being by means of electrical signals from the heart acquired by a plurality of surface electrodes being arranged on an outer surface of the living being is presented, wherein the system is adapted to be used with a vest for being worn by the living being and comprising the plurality of surface electrodes and an optical shape sensing fiber, the system comprising:
- a spatial relation providing unit for providing spatial relations between the optical shape sensing fiber of the vest and the surface electrodes,
- a surface electrodes positions calculation unit for calculating the positions of the surface electrodes depending on the three-dimensional shape of the optical shape sensing fiber as indicated by the optical shape sensing signal provided by the optical shape sensing fiber and the provided spatial relation,
- a cardiac structure position calculation unit for calculating the position of the cardiac structure based on a) the optical shape sensing signal received from the optical shape sensing fiber embedded in the vest, b) an ultrasound signal received from an ultrasound unit equipped with an optical shape sensing fiber, wherein the ultrasound signal is indicative of the position of the cardiac structure, and c) the optical shape sensing signal received from the optical shape sensing fiber of the ultrasound unit, wherein the optical shape sensing signal is indicative of the position of the ultrasound unit,
- an electrical activity map determination unit for determining the electrical activity map at the cardiac structure based on the electrical signals measured on the outer surface of the living being, the determined positions of the plurality of electrodes and the determined position of the cardiac structure.

Since the positions of the plurality of surface electrodes are determined by means of optical shape sensing localization, these positions can be determined without necessarily acquiring a computed tomography image showing the plurality of surface electrodes. This allows reducing, in particular, eliminating, the x-ray radiation dose applied to the living being for generating an electrical activity map of the heart.

The cardiac structure is preferentially the epicardial surface of the heart.

If the cardiac structure is three-dimensional, in particular, if the cardiac structure is the three-dimensional epicardial surface, the position of the cardiac structure preferentially defines the position of each point of the cardiac structure at which the electrical activity map should be determined. Thus, for instance, if the cardiac structure position determination unit determines the position of the epicardial surface, it determines at least the positions of the points on the epicardial surface for which an electrical potential should be determined for generating the electrical activity map.

The plurality of surface electrodes can be regarded as being an element of the system or it can be regarded as being a separate element, wherein the system is adapted to use the electrical signals of the surface electrodes for providing the electrical activity map.

The plurality of surface electrodes is incorporated in a vest that can be worn by the living being. The living being is preferentially a person, but the living being can also be an animal.

The system comprises preferentially the ultrasound unit for generating an ultrasound signal being indicative of the position of the cardiac structure. The ultrasound unit is preferentially a transthoracic echo probe or a transesophageal echo probe. If the ultrasound unit is a transthoracic echo probe or a transesophageal echo probe, an ultrasound image of the heart showing the epicardial surface can be acquired with high quality, thereby allowing the cardiac structure position calculation unit to determine the position of the epicardial surface being the preferred cardiac structure with high accuracy.

In an embodiment the cardiac structure position calculation unit is adapted to perform a segmentation procedure for segmenting the cardiac structure in the ultrasound image for detecting the cardiac structure. In another embodiment the cardiac structure position calculation unit is adapted to provide an anatomical cardiac model being an anatomical model of a heart including the cardiac structure and to adjust the cardiac model to the ultrasound image of the heart for detecting the cardiac structure. The adjustment can just be a rotation and/or translation and optionally a scaling of the cardiac model, or it can also include a deformation of the cardiac model. The cardiac model is preferentially a generalized cardiac model, i.e. a cardiac model which is, before being adjusted, not specific for a certain person or animal. It can be determined by, for instance, averaging segmented hearts of a group of living beings, which may be segmented in medical images.

The ultrasound unit is equipped with an optical shape sensing sensor for generating an optical shape sensing signal being indicative of the position of the ultrasound unit, wherein the cardiac structure position calculation unit is adapted to determine the position of the cardiac structure based on the optical shape sensing signal and the ultrasound signal. The optical shape sensing sensor is an optical shape sensing fiber, which may be partly arranged within the ultrasound unit. This allows determining the position of the ultrasound unit by optical shape sensing, without applying, for instance, x-rays to the person. In particular, if the ultrasound signal represents an ultrasound image of the heart, the epicardial surface in the ultrasound image can be segmented for determining the position of the epicardial surface within the ultrasound image and this determined position of the epicardial surface can be related to a reference position, i.e. it can be determined within a reference coordinate system, based on the position of the ultrasound unit known from the optical shape sensing signal.

The system can further comprise a movement determination unit for determining a movement of the living being, wherein the surface electrodes positions determination unit can be adapted to determine the positions of the plurality of surface electrodes depending on the determined movement. The movement determination unit can comprise an optical shape sensing sensor for being attached to the living being at an attachment location and for generating an optical shape sensing signal being indicative of an actual position of the optical shape sensing sensor and a movement calculation unit for calculating a movement of the living being depending on the generated optical shape sensing signal. One or several optical shape sensing sensors can be used for determining the movement of the living being. The optical shape sensing sensors can be adapted to be directly attached to the living being or to be attached to another means being attached to the living being. The other means can be, for instance, the vest comprising the plurality of the surface electrodes or patches that can be put on, for example, a person's thorax. Considering a possible movement of the person while determining the electrical activity map of the heart can reduce corresponding possible inaccuracies in the electrical activity map.

The optical shape sensing fiber is incorporated into the vest which also comprises the plurality of surface electrodes, wherein the spatial relations between the optical shape sensing fiber and the plurality of surface electrodes are known and stored in the spatial relation providing unit. This allows determining the positions of the surface electrodes without using, for instance, an optical shape sensing wand touching the different surface electrodes. For instance, an electrical activity map can be generated just by using the vest with the one or several optical shape sensing sensors and an ultrasound unit also being equipped with an optical shape sensing sensor such that the position of the ultrasound unit relative to the surface electrodes can be determined by optical shape sensing, preferentially without requiring further means for determining the positions of the surface electrodes and the cardiac structure. Since less elements are needed for generating the electrical activity map, the process for determining the electrical activity map to be performed by, for instance, a physician can be simplified.

In a further aspect of the present invention a method for providing an electrical activity map of the heart of a living being by means of electrical signals from the heart acquired by a plurality of surface electrodes being arranged on an outer surface of the living being is presented, the method comprising:
- determining positions of the plurality of surface electrodes, wherein the vest is worn by the living being and comprises the plurality of surface electrodes, wherein spatial relations between the optical shape sensing fiber of the vest and the surface electrodes are provided by a spatial relation providing unit, and wherein the positions of the surface electrodes are calculated by a surface electrodes positions calculation unit depending on the three-dimensional shape of the optical shape sensing fiber as indicated by the optical shape sensing signal provided by the optical shape sensing fiber and the provided spatial relation,
- determining a position of a cardiac structure of the living being by an cardiac structure position calculation unit, wherein the position of the cardiac structure is determined based on a) the optical shape sensing signal received from the optical shape sensing fiber embedded in the vest, b) an utrasound signal received from a transthoracic ultrasound unit equipped with an optical shape sensing fiber, wherein the ultrasound signal is indicative of the position of the cardiac structure, and c) the optical shape sensing signal received from the optical shape sensing sensor of the ultrasound unit, wherein the optical shape sensing signal is indicative of the position of the ultrasound unit,
- determining the electrical activity map at the cardiac structure by an electrical activity map determination unit based on the electrical signals measured on the outer surface of the living being, the determined positions of the plurality of electrodes and the determined position of the cardiac structure.

In a further aspect of the present invention a computer program for providing an electrical activity map of the heart of a living being by means of electrical signals from the heart acquired by a plurality of surface electrodes being arranged on an outer surface of the living being is presented, the computer program comprising program code means for causing a system as defined in claim 1 to carry out the steps of the method as defined in claim 6, when the computer program is run on a computer controlling the system.

It shall be understood that the system of claim 2, the method of claim 6 and the computer program claim 7 have similar and/or identical preferred embodiments, in particular, as defined in the dependent claims.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a system for providing an electrical activity map of the heart of a living being, which does not form part of the present invention,
Fig. 2 shows schematically and exemplarily a further embodiment of a system in accordance with the present invention for providing an electrical activity map of the heart of the living being, and
Fig. 3 shows a flowchart exemplarily illustrating an embodiment of a method for providing an electrical activity map of the heart of a living being.

### DETAILED DESCRIPTION OF EMBODIMENTS

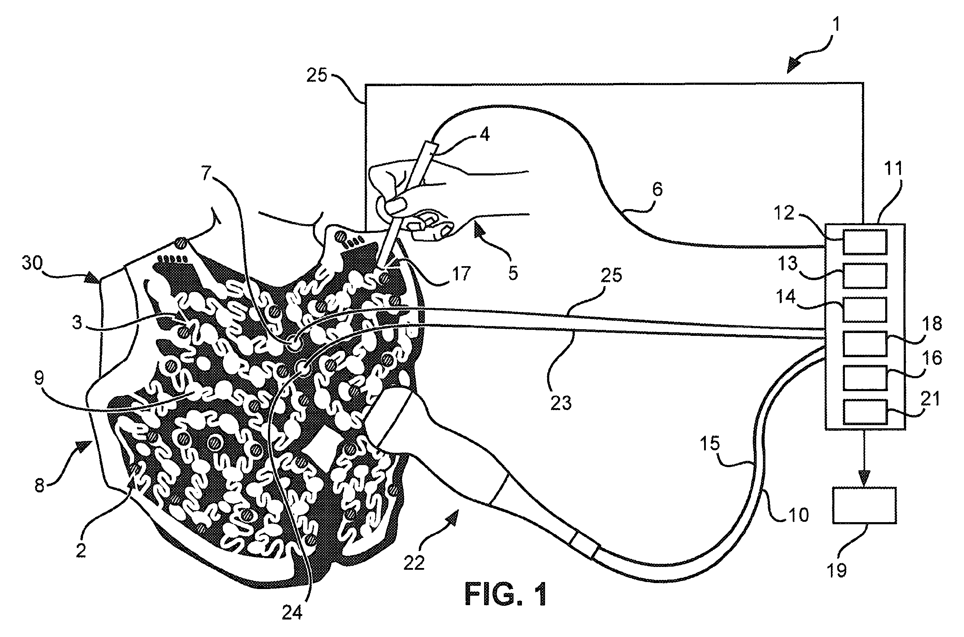

Fig. 1 shows schematically and exemplarily an embodiment of a system for providing an electrical activity map of the heart of a living being by means of electrical signals from the heart acquired by a plurality of surface electrodes being arranged on an outer surface of the living being. The system 1 comprises an optical shape sensing element 4, 6 for generating an optical shape sensing signal being indicative of the position of the tip 17 of the optical shape sensing element 4, 6 while being in contact with a respective reference mark 2, in order to generate an optical shape sensing signal being indicative of the position of the respective reference mark 2. In particular, the living being 30 being, in this embodiment, a person wears a vest 8 with surface electrodes 9 and reference marks 2. A user like a physician can use the optical shape sensing element 4, 6 such that the tip of the optical shape sensing element 4, 6 consecutively touches the different reference marks 2 of the vest 8, in order to determine the positions of the reference marks 2. The vest 8 is electrically connected with a determination system 11 via an electrical connection 25, in order to transmit the electrical signals acquired by the surface electrodes 9 to the electrical activity map determination unit 16.

The optical shape sensing element 4, 6 comprises a wand 4, which can be held by a hand 5 of a user, with the tip 17 for being brought in contact with different reference marks 2 and an optical shape sensing fiber 6 being connected to the wand 4. The optical shape sensing element 4, 6 can be adapted to, for example, continuously generate an optical shape sensing signal or to generate an optical shape sensing signal only after a user has requested an optical shape sensing signal via an input unit like a button to be pressed. The tip can also be provided with a pressure sensitive sensor for detecting whether the tip is in contact with an element or not, wherein the optical shape sensing element can be adapted to generate an optical shape sensing signal, when the pressure sensitive sensor detects that the tip is in contact with a reference mark.

The system 1 comprises a further optical shape sensing element, i.e. an optical shape sensing sensor 7, 26 for being attached to the person 30 at an attachment location and for generating an optical shape sensing signal being indicative of the actual position of the optical shape sensing sensor 7, 26. In this embodiment this optical shape sensing sensor comprises a reference patch 7 connected to an optical shape sensing fiber 26, wherein the generated optical shape sensing signal is indicative of the position of the reference patch 7.

The optical shape sensing signal from the optical shape sensing sensor 7, 26, which is indicative of the position of the reference patch 7, and the optical shape sensing signal from the optical shape sensing element 4, 6, which is indicative of the position of the tip 17 of the optical shape sensing element 4, 6, are provided to a surface electrodes positions calculation unit 13 of the determination system 11. The surface electrodes positions calculation unit 13 is adapted to determine the three-dimensional positions of the reference marks 2 with respect to the reference patch 7 based on the provided optical shape sensing signals. For determining the positions of the reference marks 2 known optical shape sensing localization method can be used like the optical shape sensing methods disclosed in WO 2011/141830 A1.

After the positions of the reference markers 2 have been determined, the surface electrodes positions calculation unit 13 determines the positions of the surface electrodes 9 depending on the determined positions of the reference marks 2 and known spatial relations between the positions of the plurality of surface electrodes 9 and the positions of the reference marks 2 provided by a spatial relation providing unit 12.

Thus, the wand 4 with the optical shape sensing fiber 6 is used to measure the positions of the reference marks 2 in the three-dimensional space with respect to the reference patch 7. By going from one reference mark 2 to another reference mark 2 and recording the three-dimensional positions, a three-dimensional distribution of the reference marks 2 and thus, since the spatial relations between the reference marks 2 and the surface electrodes 9 being connected by tortuous wires 3 are known, the three-dimensional distribution of the vest electrodes can be reconstructed. The wand 4 with the optical shape sensing fiber 6, the spatial relations providing unit 12 and the surface electrodes positions calculation 13 can therefore be regarded as being components of a surface electrodes positions determination unit for determining the positions of a plurality of surface electrodes by means of optical shape sensing localization.

The system 1 further comprises an ultrasound unit 22 for generating an ultrasound signal being indicative of the position of the cardiac structure within the person 30. The ultrasound signal is provided to the determination system 11 via the electrical connection 15. In this embodiment the ultrasound unit is a transthoracic echo probe 22 for generating an ultrasound signal representing a three-dimensional ultrasound image of the heart. In another embodiment the ultrasound unit can also be another probe like a transesophageal echo probe.

The ultrasound signal is provided to a cardiac structure position calculation unit 14 for calculating the position of the cardiac structure based on the ultrasound signal. In particular, the cardiac structure calculation unit 14 is adapted to detect the cardiac structure in the ultrasound image and to calculate the position of the cardiac structure based on the cardiac structure detected in the ultrasound image. For instance, the cardiac structure position calculation unit 14 can be adapted to perform a segmentation procedure for segmenting the cardiac structure being, in this embodiment, the epicardial surface, in the ultrasound image for detecting the cardiac structure. The cardiac structure position calculation unit can also be adapted to provide an anatomical cardiac model being an anatomical model of a heart including the cardiac structure, i.e. in this embodiment including the epicardial surface, and to adjust the cardiac model to the ultrasound image of the heart for detecting the cardiac structure. The cardiac model is preferentially a generalized cardiac model, i.e. a cardiac model which is, before being adjusted, not specific for a certain person or animal. It can be determined by, for instance, averaging of segmented hearts of a group of living beings, which may be segmented in medical images. In order to allow the cardiac structure position calculation unit to calculate the position of the detected cardiac structure, the cardiac structure position calculation unit 14 further receives an optical shape sensing signal from the ultrasound unit 22. The ultrasound unit 22 is equipped with an optical shape sensing sensor 10 for generating the optical shape sensing signal being indicative of the position of the ultrasound unit 22, wherein the cardiac structure position calculation unit 14 is adapted to determine the position of the ultrasound unit 22 with respect to the position of the reference patch 7 based on the optical sensing signals received from the optical shape sensing sensor 10 of the ultrasound unit 22, which is an optical shape sensing fiber, and from the optical shape sensing sensor comprising the reference patch 7 and the optical shape sensing fiber 26 connected to the patch 7. The position of the cardiac structure, i.e. in this embodiment of the epicardial surface, is then determined based on the determined three-dimensional position of the ultrasound unit 22 and the determined position of the cardiac structure within the ultrasound image acquired by the ultrasound unit 22.

Thus, an ultrasound probe 22 with an optical shape sensing fiber 10 is used to image the cardiac anatomy, wherein the optical shape sensing fiber 10, which is preferentially embedded in the ultrasound probe 22, is used to measure the location of the ultrasound probe 22 with respect to the reference patch 7, thus ensuring that the location of the cardiac anatomy as imaged by the ultrasound probe 22 and the positions of the surface electrodes 9 in three-dimensional space are known with respect to each other. In other words, since in this embodiment the positions of the surface electrodes 9 and of the epicardial surface are determined with respect to the same reference being the reference patch 7, the spatial relation between the surface electrodes 9 and the epicardial surface is known. In other embodiments, the positions of the surface electrodes and the position of, for instance, the epicardial surface can be determined with respect to another reference. The optical shape sensing sensor 10, the cardiac structure position calculation unit 14 and the ultrasound unit 22 with the electrical connection 15 for transferring the ultrasound signal can be regarded as being elements of a cardiac structure position determination unit for determining a position of a cardiac structure like the epicardial surface of the person 30.

The system further comprises a movement determination unit for determining a movement of the person 30, wherein the surface electrodes positions calculation unit 13 is adapted to determine the positions of the plurality of surface electrodes 9 also depending on the determined movement. The movement determination unit comprises a further patch 24, a further optical shape sensing fiber 23 attached to the patch 24 and a movement calculation unit 18. The movement calculation unit 18 receives an optical shape sensing signal being indicative of the position of the further patch 24 attached to the vest 8, wherein for providing this optical shape sensing signal the patch 24 is connected with the optical shape sensing fiber 23. The movement calculation unit 18 calculates the position of the patch 24 at different times from the received optical shape sensing signal, in order to determine the movement of the person 30.

One or several optical shape sensing sensors, for instance, one or several patches with optical shape sensing fibers, can be used for determining the movement of the person 30. The optical shape sensing sensors for determining the movement of the person can be adapted to be directly attached to the person. For instance, the optical shape sensing sensors, in particular, patches connected to optical shape sensing fibers, can be put on a person's thorax. Alternatively or in addition, the optical shape sensing sensors can be attached to another means being attached to the person. The other means can be, as shown in Fig. 1, a vest comprising the plurality of surface electrodes.

The system 1 further comprises an electrical activity map determination unit 16 for determining the electrical activity map at the cardiac structure, i.e. in this embodiment on the epicardial surface, based on the electrical signals measured on the outer surface of the person 30, the determined positions of the plurality of electrodes 9 and the determined position of the cardiac structure. For determining the electrical activity map well known methods can be used like the methods disclosed in the article "Electrocardiographic Imaging (ECGI): A Noninvasive Imaging Modality for Cardiac Electrophysiology and Arrhythmia" by Ramanathan et al., Nature Medicine 10, 422-428 (2004) or disclosed in US 7,471,973. Moreover, known products from the companies CardioInsight Technologies and Amycard can be used for determining the electrical activity map at the cardiac structure, i.e. in this embodiment on the epicardial surface, based on the electrical signals measured on the outer surface of the person, the determined positions of the plurality of electrodes and the determined position of the cardiac structure.

The determination system 11 further comprises an analysis unit 21 for analyzing the electrical activity map for determining electrophysiological mechanisms of certain cardiac arrhythmias. Moreover, in addition or alternatively the analysis unit 21 may be adapted to analyze the electrical activity behaviour of cardiac dyssnychrony in heart failure patients as disclosed in the articles "Noninvasive Characterization of Epicardial Activation in Humans with Diverse Atrial Fibrillation Patterns" by P.S. Cuculich et al., Circulation 122, 1364-1372 (2010), "Electrocardiographic Imaging of Ventricular Bigeminy in a Human Subject" by Y. Wang et al., Circulation Arrhythmia and Electrophysiology 1, 74-75 (2008) and "Electrocardiographic Imaging of Cardiac Resynchronization Therapy in Heart Failure: Observations of Variable Electrophysiological Responses" by P. Jia et al., Heart Rhythm Journal 3, 296-310 (2006).

In particular, the analysis unit can be adapted to perform at least one of following analyses based on the electrical activity map: determination of an anatomical position of ectopi foci, determination of an anatomical position of ventricular re-entries, distinguishing between re-entry or focal ventricular tachycardia and assessing of its localizations, assessing re-connection of pulmonary vein conduction and localization of culprit pulmonary veins, and assessment of effects of antiarrhythmic drugs.

The electrical activity map of the heart and optionally results of the analysis can be shown on a display unit 19.

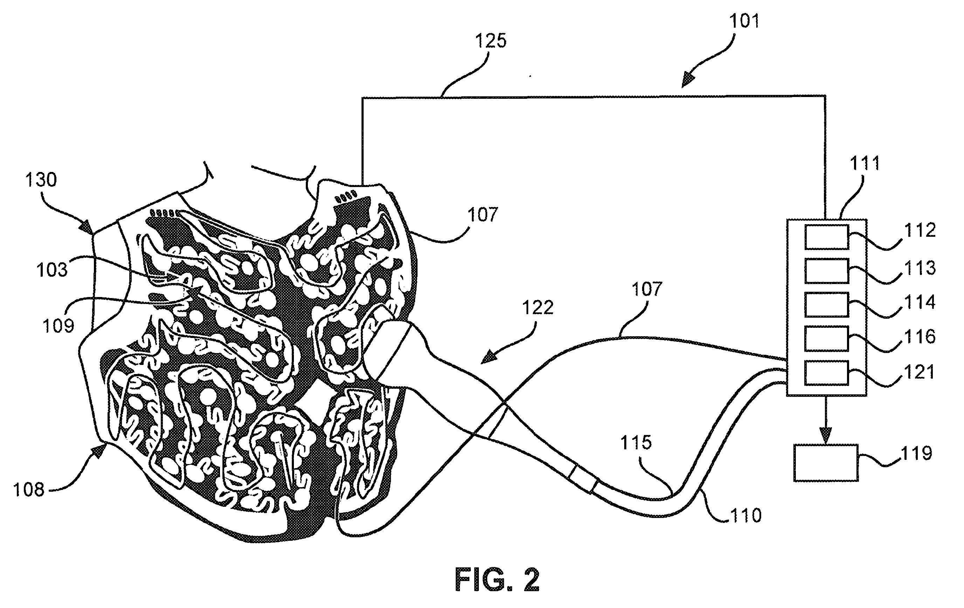

Fig. 2 shows schematically and exemplarily a further embodiment of a system for providing an electrical activity map of the heart of a living being by means of electrical signals from the heart acquired by a plurality of surface electrodes being arranged on an outer surface of the living being. Also in this embodiment the living being is a person 130 wearing a vest 108 with a plurality of electrodes 109 connected by tortuous wires 103. The surface electrodes 109 are used for acquiring electrical signals at the outer surface of the person 130, wherein the acquired electrical signals are provided to a determination system 111 via an electrical connection 125.

The vest 108 comprises an optical shape sensing fiber 107 for providing an optical shape sensing signal being indicative of the position of each portion of the optical shape sensing fiber 107 within the vest 108. An ultrasound unit 122 with an optical shape sensing fiber 110 and an electrical connection 115 for providing ultrasound signals to the determination system 111 is used for generating a three-dimensional ultrasound image of the heart of the person 130. The ultrasound unit 122 with the optical shape sensing fiber 110 and the electrical connection 115 is similar to the ultrasound unit 22 with the optical shape sensing fiber 10 and the electrical connection 15 described above with reference to Fig. 1.

The determination system 111 comprises a spatial relation providing unit 112 for providing a spatial relation between the optical shape sensing fiber 107 within the vest 108 and the surface electrodes 109. This spatial relation is used together with the optical shape sensing signal, which is provided by the optical shape sensing fiber 107 and which is indicative of the position of each portion of the optical shape sensing fiber 107 within the vest 108, for determining the position of the surface electrodes 109 incorporated into the vest 108. Thus, the shape of at least one optical shape sensing fiber 107, which is embedded in a specific pattern in the vest 108, can be measured for determining the position of each portion of the optical shape sensing fiber 107 within the vest 108. The locations of the surface electrodes 109 with respect to the optical shape sensing fiber 107 are known from the spatial relation provided by the spatial relation providing unit 112. Therefore, a surface electrodes positions calculation unit 113 can calculate the three-dimensional distribution of the surface electrodes 109 by measuring the three-dimensional shape of the optical fiber 107. The optical shape sensing fiber 107, the spatial relation providing unit 112 and the surface electrodes positions calculation unit 113 can therefore be regarded as being elements of a surface electrodes positions determination unit for determining positions of the plurality of surface electrodes 109 by means of optical shape sensing localization.

The determination system 111 further comprises a cardiac structure position calculation unit 114 for calculating the position of the cardiac structure, i.e. in this embodiment of the epicardial surface, based on the optical shape sensing signal received from the vest optical shape sensing fiber 107, the optical shape sensing signal received from the ultrasound optical shape sensing fiber 110 and the ultrasound signal acquired by the ultrasound unit 122. In particular, the ultrasound signal is a three-dimensional ultrasound image of the heart of the person 130, wherein the cardiac structure position calculation unit 114 is adapted to segment the epicardial surface in the three-dimensional ultrasound image for determining the position of the epicardial surface within this image. The position of this epicardial surface with respect to the position of the surface electrodes 109 is then determined by determining the position of the ultrasound unit 122 with respect to the position of the optical shape sensing fiber 107. Thus, the ultrasound unit 122, which can be regarded as being an ultrasound probe wand, is used to image the cardiac anatomy, wherein the optical shape sensing fiber 110, which is embedded in the ultrasound unit 122, is used to measure the location of the ultrasound unit 122 with respect to the optical shape sensing fiber 107 embedded in the vest 108, thereby ensuring that the location of the cardiac anatomy, i.e. in this embodiment of the epicardial surface, as imaged by the ultrasound unit 122 and the position of the surface electrodes 109 are known in the three-dimensional space. The optical shape sensing fiber 110, the cardiac structure calculation determination unit 114 and the ultrasound unit 122 with the electrical connection 115 for transferring the ultrasound signals can be regarded as being elements of a cardiac structure position determination unit for determining a position of a cardiac structure of the person 130.

The determination system 111 further comprises an electrical activity map determination unit 116 and an analysis unit 121, which are similar to the electrical activity map determination unit 16 and the analysis unit 21, respectively, described above with reference to Fig. 1. Also the display unit 119 is similar to the display unit 19 described above with reference to Fig. 1.

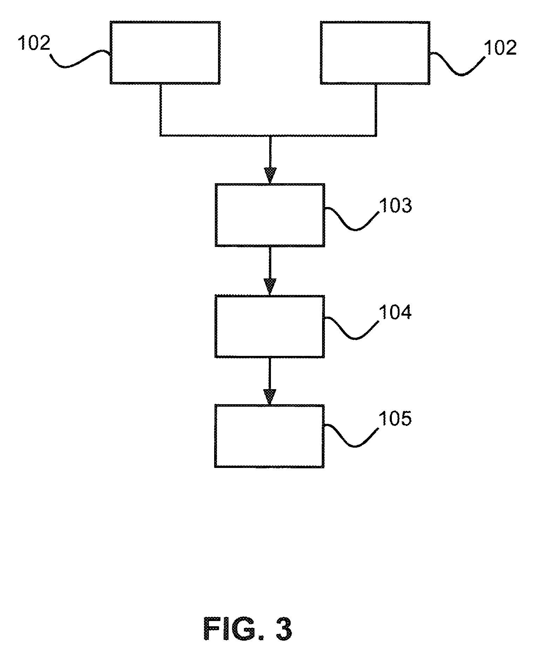

In the following a method for providing an electrical activity map of the heart of a living being by means of electrical signals from the heart acquired by a plurality of surface electrodes at an outer surface of the living being will exemplarily be described with reference to a flowchart shown in Fig. 3.

In step 101, the positions of the plurality of surface electrodes are determined by the surface electrodes positions determination unit by means of optical shape sensing localization. For instance, the wand 4 with the connected optical shape sensing fiber 6 described above with reference to Fig. 1 is used for determining the three-dimensional positions of reference marks of a vest worn by a person by using optical shape sensing. A surface electrodes positions calculation unit can then calculate the three-dimensional positions of the electrodes of the vest based on the determined three-dimensional positions of the reference marks and provided spatial relations between the reference marks and the surface electrodes incorporated in the vest. Alternatively, an optical shape sensing fiber 107 embedded in the vest worn by the person can be used for determining the three-dimensional position of the surface electrodes as described above with reference to Fig. 2. In particular, the three-dimensional position of each portion of the optical shape sensing fiber 107 within the vest 108 can be determined by optical shape sensing, wherein the surface electrodes positions calculation unit can calculate the three-dimensional positions of the surface electrodes based on the determined three-dimensional position of each portion of the optical shape sensing fiber 107 within the vest 108 and a provided spatial relation between the optical shape sensing fiber within the vest and the surface electrodes embedded in the vest.

In step 102, the position of a cardiac structure of the person 30 is determined. In this embodiment the position of the epicardial surface is determined. For instance, the ultrasound unit with the optical shape sensing sensor described above with reference to Figs. 1 and 2 is used for generating a three-dimensional ultrasound image showing the epicardial surface, wherein the cardiac structure position calculation unit can calculate the three-dimensional position of the epicardial surface based on, for example, a segmentation of the epicardial surface in the three-dimensional ultrasound image and the position of the ultrasound unit determined by optical shape sensing localization. Steps 101 and 102 can be performed in an arbitrary order, i.e. they can be performed consecutively or simultaneously.

In step 103, the electrical activity map determination unit determines the electrical activity map at the cardiac structure, i.e., in this embodiment, on the epicardial surface, based on the electrical signals measured on the outer surface of the person, the positions of the plurality of surface electrodes determined in step 101 and the position of the cardiac structure determined in step 102. In step 104, the analysis unit analyzes the electrical activity map, in order to determine, for example, electrophysiological mechanisms of certain cardiac arrhythmias. In step 105, the electrical activity map and optionally also results of the analysis are shown on the display unit.

In the embodiment of the method for providing an electrical activity map of the heart described above with reference to Fig. 3 it is assumed that the electrical signals on the outer surface of the person have been measured already and are provided to the electrical activity map determination unit for allowing the electrical activity map determination unit to determine the electrical activity map. In another embodiment the measurement of the electrical signals on the outer surface of the person can also be a part of the method for providing the electrical activity map, wherein in this case a corresponding electrical signals measurement step is performed before step 103.

Electrocardiographic mapping (ECM) is a method where body surface signals, i.e. electrical signals like electrical potentials measured at the outer surface of the person, which are measured by a multitude of electrodes covering the entire human thorax, are used to calculate the activation of the epicardial surface of the heart. The electrodes are surface electrodes, i.e. electrodes measuring electrical signals at the surface of the person, and they are contained in the vest that is tightly attached to the skin of the thorax by an adhesive. Alternatively or in addition, the vest can comprise elastic textiles for tightly fitting the vest to the skin of the thorax. Since the position of the epicardial heart surface and the positions of the surface electrodes have been determined, the three-dimensional spatial relations between the epicardial heart surface and the surface electrodes are known. This enables the electrical activity map determination unit to compute an accurate single beat electrical activation pattern on the epicardial heart surface being the electrical activity map. The system described above with reference to Fig. 1 can therefore provide a non-invasive method for rapid assessment, i.e. within seconds to real time, of cardiac electrical activation. This electrocardiographic mapping can be performed, for example, during an electrophysiological procedure or during interventional cardiology procedures in a corresponding laboratory. However, the electrocardiographic mapping can also be performed for pre-interventional or post-interventional follow-up diagnostic procedures. In particular, the electrocardiographic mapping can be used to assess effects of antiarrhythmic drugs at certain points during a course of drug use and may be used for performing a high resolution electrocardiographic analysis.

The systems described above with reference to Figs. 1 and 2 allow a rapid assessment of the three-dimensional positions of the vest electrodes, i.e. of the surface electrodes, by means of optical shape sensing localization and the assessment of the three-dimensional cardiac anatomy in relation to the three-dimensional positions of the vest electrodes by means of a transthoracic or transesophageal echo probe that is localized in the three-dimensional space by means of optical shape sensing localization. The systems can be used as an advanced electrocardiography tool with epicardial activation diagnosis capabilities.

Preferentially, the configuration of the electrodes in the electrocardiography mapping vest within the vest fabric is known. In an embodiment, in particular in the embodiment described above with reference to Fig. 1, within the vest are a plurality of landmark points, i.e. reference marks, that can be visually identified, for instance, by having a certain color and/or shape. The positions of electrodes in relation to the landmark points are known as well. After putting on the vest to the person the three-dimensional positions of the landmark points can be determined by touching the landmarks by means of the wand equipped with the optical shape sensing sensor. The number and distribution of the landmark points are preferentially such that the relative positions of all electrodes in relation to the landmarks and therefore in relation to a frame of reference can be calculated based on the known positions of the electrodes within the fabric and in relation to the landmarks. To avoid person motion distortion, one or more additional optical shape sensing sensors can be temporally added to the vest or attached to patches that are put somewhere on the patient's thorax to compensate for the person movement.

Preferentially, the ultrasound unit is used for reconstructing a transthoracic or transesophageal three-dimensional representation of the person's cardiac anatomy. This reconstruction can be based on three-dimensional imaging of the heart and subsequent segmentation of the cardiac structures or by matching a generalized three-dimensional cardiac model to the three-dimensional ultrasound image, wherein optionally the generalized three-dimensional cardiac model can be deformed. The ultrasound probe is equipped with optical shape sensing localization as well, thus allowing to correlate the three-dimensional cardiac anatomy with the vest electrode positions. In an embodiment the ultrasound based cardiac anatomy assessment is performed before or after the vest electrodes positions assessment. In both cases optical shape sensing sensors temporally added to the vest or attached to a patch that is put somewhere on the patient's thorax can be used to relate the ultrasound probe position and therefore the three-dimensional cardiac anatomy to the vest electrode positions.

In an embodiment, in particular in the embodiment described above with reference to Fig. 2, the electrode vest can be equipped with one or more optical shape sensing fibers, wherein the positions of the vest electrodes are identified and therefore known in relation to the one or more optical shape sensing fibers. Through assessing the three-dimensional shape of the one or more optical shape sensing fibers the three-dimensional positions of the vest electrodes can be calculated. Also in this embodiment the position of the cardiac anatomy is determined as described above by using an ultrasound probe, wherein in this embodiment the position of the ultrasound probe is registered with the position of the vest electrodes by using the optical shape sensing signals from the one or more optical shape sensing fibers within the vest and from the optical shape sensing sensor of the ultrasound probe.

Although in the embodiment described above with reference to Fig. 1 an additional wand 4 equipped with an optical shape sensing fiber 6 is used for determining the three-dimensional positions of reference marks 2, in another embodiment this additional wand 4 with the optical shape sensing fiber 6 is not needed. Instead, the ultrasound probe 22 can be used for determining the three-dimensional positions of the reference marks 2. Thus, a user like a physician can move the ultrasound probe 22 from reference mark to reference mark, in order to determine the three-dimensional positions of these reference mark by optical shape sensing localization. These three-dimensional positions of the reference marks are then used by the surface electrodes position calculation unit for determining the three-dimensional positions of the surface electrodes as described above. The same ultrasound probe 22 can then be used for providing an ultrasound signal being indicative of the cardiac structure, in particular, being indicative of the epicardial surface, in order to determine the position of the epicardial surface.

The ultrasound probe is preferentially used through an opening in the vest directly on the skin of the thorax, or before the vest is put on by the person. In the embodiment described above with reference to Fig. 1, the ultrasound probe 22 may be used on the skin of the thorax for acquiring an ultrasound image of the heart before the vest is put on by the person, but after a reference patch being connected to an optical shape sensing fiber for determining the position of the reference patch is applied to the skin of the thorax.

The locations of the surface electrodes of the vest can be determined by means of Fiber Optic Shape Sensing and Localization (FOSSL) technology, for example, based on Fiber Bragg Gratings. The vest may contain one or more fibers with such grating that allow the full three-dimensional shape and location of the fibers to be determined. By knowing the spatial relation between each of the surface electrodes and a relevant fiber, the location of each of the electrodes can be determined. This can then be realized without any x-ray or magnetic resonance based localization of the surface electrodes. Moreover, the ultrasound probe can be a three-dimensional transesophageal or micro transthoracic ultrasound probe which can be used to reconstruct the heart. Furthermore, the FOSSL technology can be used to localize the probe by means of such a fiber, in particular, in a catheter. The FOSSL technology can be used to localize the surface electrodes as described above.

The systems described above with reference to Figs. 1 and 2 provide an electrocardiographic mapping based on a three-dimensional assessment of vest electrode positions by means of optical shape sensing localized vest electrodes and based on a three-dimensional cardiac anatomy assessment of optical shape sensing localized ultrasound imaging. The system can therefore provide an electrocardiographic diagnostic tool that can be used for obtaining, for instance, pre-interventional and post-interventional information that is not obtainable by known electrocardiographic systems. For example, information can be provided such as reasonably accurate positions of ectopic foci, reasonable accurate positions of ventricular re-entries, information that distinguishes between re-entry or focal ventricular tachycardia and its localizations, information about re-connection of pulmonary vein conduction and localization of culprit pulmonary veins, in order to be at least able to distinguish between left and right pulmonary veins, and information about effects of antiarrhythmic drugs, in particuluar, of changes in use of antiarrhythmics drugs.

Although in above described embodiments, in particular, in Figs. 1 and 2, the vest has a certain distribution of electrodes, it should be noted that the electrodes in the vest are only schematically and exemplarily indicated in Figs. 1 and 2, i.e., for instance, they can be distributed differently within the vest. The vest preferentially comprises several hundreds of the electrodes covering the entire thorax of the person.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the calculation of the surface electrodes positions, the calculation of the cardiac structure position, the calculation of the electrical activity map and/or the analysis of the electrical activity map performed by one or several units or devices can be performed by any other number of units or devices. The calculations and/or the analysis of the electrical activity map and/or the control of the system for providing an electrical activity map in accordance with the method for providing an electrical activity map can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a system for providing an electrical activity map of the heart of a living being by means of electrical signals from the heart acquired by a plurality of surface electrodes being arranged on an outer surface of the living being. A surface electrodes positions determination unit determines positions of the plurality of surface electrodes by means of optical shape sensing localization and an electrical activity map determination unit determines the electrical activity map at the cardiac structure based on the measured electrical signals, the determined positions of the plurality of electrodes and a position of a cardiac structure, in particular, of the epicardial surface. Since optical shape sensing is used for determining the positions of the plurality of surface electrodes and not, for instance, x-rays, the electrical activity map can be determined, without necessarily applying an x-ray radiation dose.

## Claims

1. A vest for being worn by a living being, the vest being adapted to be used for providing an electrical activity map of the heart of a living being (30), the vest (108) comprising:
- a plurality of surface electrodes (109) for being arranged on an outer surface of the living being (30), when the vest (108) is worn by the living being (30), and for acquiring electrical signals from the heart of the living being (30),
- an optical shape sensing fiber (107) for generating an optical shape sensing signal being indicative of the three-dimensional shape of the optical shape sensing fiber (107) and for providing the optical shape sensing signal to a surface electrodes positions calculation unit.

2. A system for providing an electrical activity map of the heart of a living being by means of electrical signals from the heart acquired by a plurality of surface electrodes being arranged on an outer surface of the living being, wherein the system is adapted to be used with a vest as defined in claim 1 for being worn by the living being (30) and comprising the plurality of surface electrodes, the system comprising:
- a spatial relation providing unit (112) for providing spatial relations between the optical shape sensing fiber (107) of the vest (108) and the surface electrodes (109),
- a surface electrodes positions calculation unit (113) for calculating the positions of the surface electrodes (109) depending on the three-dimensional shape of the optical shape sensing fiber (107) as indicated by the optical shape sensing signal provided by the optical shape sensing fiber (107) and the provided spatial relation,
- a cardiac structure position calculation unit (114) for calculating the position of a cardiac structure of the living being based on a) the optical shape sensing signal received from the optical shape sensing fiber (107) embedded in the vest, b) an ultrasound signal received from an ultrasound unit (122) equipped with an optical shape sensing fiber (110), wherein the ultrasound signal is indicative of the position of the cardiac structure, and c) the optical shape sensing signal received from the optical shape sensing fiber (110) of the ultrasound unit (122), wherein the optical shape sensing signal is indicative of the position of the ultrasound unit (122),
- an electrical activity map determination unit (116) for determining the electrical activity map at the cardiac structure based on the electrical signals measured on the outer surface of the living being (30), the determined positions of the plurality of electrodes (109) and the determined position of the cardiac structure.

3. The system as defined in claim 2, wherein the system comprises the ultrasound unit (122) for generating the ultrasound signal being indicative of the position of the cardiac structure, wherein the ultrasound unit (122) is equipped with the optical shape sensing sensor (110) for generating the optical shape sensing signal being indicative of the position of the ultrasound unit (122).

4. The system as defined in claim 3, wherein the ultrasound unit (122) is a transthoracic echo probe or a transesophageal echo probe.

5. The system as defined in claim 3, wherein the ultrasound signal represents an ultrasound image and wherein the cardiac structure calculation unit (14) is adapted to detect the cardiac structure in the ultrasound image and to calculate the position of the cardiac structure based on the cardiac structure detected in the ultrasound image.

6. A method for providing an electrical activity map of the heart of a living being by means of electrical signals from the heart acquired by a plurality of surface electrodes being arranged on an outer surface of the living being, the method comprising:
- determining positions of the plurality of surface electrodes, wherein a vest (108) as defined in claim 1 is worn by the living being (30) and comprises the plurality of surface electrodes (109), wherein spatial relations between the optical shape sensing fiber (107) of the vest (108) and the surface electrodes (109) are provided by a spatial relation providing unit (112), and wherein the positions of the surface electrodes (109) are calculated by a surface electrodes positions calculation unit (113) depending on the three-dimensional shape of the optical shape sensing fiber (107) as indicated by the optical shape sensing signal provided by the optical shape sensing fiber (107) and the provided spatial relation,
- determining a position of a cardiac structure of the living being by an cardiac structure position calculation unit (114), wherein the position of the cardiac structure is determined based on a) the optical shape sensing signal received from the optical shape sensing fiber (107) embedded in the vest, b) an ultrasound signal received from a transthoracic ultrasound unit (122) equipped with an optical shape sensing of fiber (110), wherein the ultrasound signal is indicative of the position of the cardiac structure, and c) the optical shape sensing signal received from the optical shape sensing sensor (110) of the ultrasound unit (122), wherein the optical shape sensing signal is indicative of the position of the ultrasound unit (122),
- determining the electrical activity map at the cardiac structure by an electrical activity map determination unit based on the electrical signals measured on the outer surface of the living being, the determined positions of the plurality of electrodes and the determined position of the cardiac structure.

7. A computer program for providing an electrical activity map of the heart of a living being by means of electrical signals from the heart acquired by a plurality of surface electrodes being arranged on an outer surface of the living being, the computer program comprising program code means for causing a system as defined in claim 2 to carry out the steps of the method as defined in claim 6, when the computer program is run on a computer controlling the system.

## Patentansprüche

1. Weste, die durch ein Lebewesen getragen werden kann, wobei die Weste dafür vorgesehen ist, eine Karte der elektrischen Aktivität des Herzens eines Lebewesens (30) bereitzustellen, wobei die Weste (108) Folgendes umfasst:
- eine Vielzahl von Oberflächenelektroden (109) zur Anordnung an einer äußeren Oberfläche des Lebewesens (30), wenn die Weste (108) durch das Lebewesen (30) getragen wird, und zum Erfassen elektrischer Signale von dem Herz des Lebewesens (30),
- einen faseroptischen Formsensor (107) zum Erzeugen eines optischen Formsensorsignals, das eine dreidimensionale Form des faseroptischen Formsensors (107) angibt, und zum Weiterleiten des optischen Formsensorsignals an eine Oberflächenelektrodenpositions-Berechnungseinheit.

2. System zur Bereitstellung einer Karte der elektrischen Aktivität des Herzens eines Lebewesens mit Hilfe der elektrischen Signale von dem Herz, die durch eine Vielzahl von Oberflächenelektroden auf der äußeren Oberfläche des Lebewesens erfasst werden, wobei das System dafür vorgesehen ist, mit einer Weste nach Anspruch 1 verwendet zu werden, die durch das Lebewesen (30) getragen werden kann und die Vielzahl von Oberflächenelektroden umfasst, wobei das System Folgendes umfasst:
- eine Raumbeziehungs-Bereitstellungseinheit (112) zum Bereitstellen der räumlichen Beziehungen zwischen dem faseroptischen Formsensor (107) der Weste (108) und den Oberflächenelektroden (109),
- eine Oberflächenelektroden-Positionsberechnungseinheit (113) zum Berechnen der Positionen der Oberflächenelektroden (109) in Abhängigkeit von der dreidimensionalen Form des faseroptischen Formsensors (107), wie sie durch das optische Formsensorsignal angegeben wird, das durch den faseroptischen Formsensor (107) bereitgestellt wird, und von der bereitgestellten räumlichen Beziehung,
- eine Herzstruktur-Positionsberechnungseinheit (114) zum Berechnen der Position einer Herzstruktur des Lebewesens basierend auf a) dem optischen Formsensorsignal des von dem in die Weste eingebetteten faseroptischen Formsensors (107), b) einem von einer Ultraschalleinheit (122), die mit einem faseroptischen Formsensor (110) ausgestattet ist, empfangenen Ultraschallsignal, wobei das Ultraschallsignal die Position der Herzstruktur angibt, und c) dem optischen Formsensorsignal von dem faseroptischen Formsensor (110) der Ultraschalleinheit (122), wobei das optische Formsensorsignal die Position der Ultraschalleinheit (122) angibt,
- eine elektrische Aktivitätskarten-Ermittlungseinheit (116) zum Ermitteln der Karte der elektrischen Aktivität an der Herzstruktur basierend auf den elektrischen Signalen, die auf der äußeren Oberfläche des Lebewesens (30) gemessen wurden, den ermittelten Positionen der Vielzahl von Oberflächenelektroden (109) und der ermittelten Position der Herzstruktur.

3. System nach Anspruch 2, wobei das System die Ultraschalleinheit (122) zum Erzeugen des Ultraschallsignals umfasst, das die Position der Herzstruktur angibt, wobei die Ultraschalleinheit (122) mit dem faseroptischen Formsensor (110) zum Erzeugen des optischen Formsensorsignals ausgestattet ist, das die Position der Ultraschalleinheit (122) angibt.

4. System nach Anspruch 3, wobei die Ultraschalleinheit (122) eine transthorakale Echosonde oder eine transösophageale Echosonde ist.

5. System nach Anspruch 3, wobei das Ultraschallsignal ein Ultraschallbild darstellt und wobei die Herzstruktur-Berechnungseinheit (14) dafür vorgesehen ist, die Herzstruktur in dem Ultraschallbild zu detektieren und die Position der Herzstruktur basierend auf der in dem Ultraschallbild detektierten Herzstruktur zu berechnen.

6. Verfahren zur Bereitstellung einer Karte der elektrischen Aktivität des Herzens eines Lebewesens mit Hilfe der elektrischen Signale von dem Herz, die durch eine Vielzahl von Oberflächenelektroden auf der äußeren Oberfläche des Lebewesens erfasst werden, wobei das Verfahren Folgendes umfasst:
- Ermitteln der Positionen der Vielzahl von Oberflächenelektroden, wobei eine Weste (108) nach Anspruch 1 durch das Lebewesen (30) getragen wird und die Vielzahl von Oberflächenelektroden (109) umfasst, wobei räumliche Beziehungen zwischen dem faseroptischen Formsensor (107) der Weste (108) und den Oberflächenelektroden (109) durch eine Raumbeziehungs-Bereitstellungseinheit (112) bereitgestellt werden, und wobei die Positionen der Oberflächenelektroden (109) durch eine Oberflächenelektrodenpositions-Berechnungseinheit (113) in Abhängigkeit von der dreidimensionalen Form des faseroptischen Formsensors (107), wie sie durch das optische Formsensorsignal angegeben wird, das durch den faseroptischen Formsensor (107) bereitgestellt wird, und von der bereitgestellten räumlichen Beziehung berechnet werden,
- Ermitteln einer Position einer Herzstruktur des Lebewesens durch eine Herzstruktur-Positionsberechnungseinheit (114), wobei die Position der Herzstruktur basierend auf a) dem optischen Formsensorsignal des von dem in die Weste eingebetteten faseroptischen Formsensors (107), b) einem von einer transthorakalen Ultraschalleinheit (122), die mit einem faseroptischen Formsensor (110) ausgestattet ist, empfangenen Ultraschallsignal, wobei das Ultraschallsignal die Position der Herzstruktur angibt, und c) dem optischen Formsensorsignal von dem faseroptischen Formsensor (110) der Ultraschalleinheit (122), wobei das optische Formsensorsignal die Position der Ultraschalleinheit (122) angibt, berechnet wird,
- Ermitteln der Karte der elektrischen Aktivität an der Herzstruktur durch eine elektrische Aktivitätskarten-Ermittlungseinheit basierend auf den elektrischen Signalen, die auf der äußeren Oberfläche des Lebewesens gemessen wurden, den ermittelten Positionen der Vielzahl von Oberflächenelektroden und der ermittelten Position der Herzstruktur.

7. Computerprogramm zur Bereitstellung einer Karte der elektrischen Aktivität des Herzens eines Lebewesens mit Hilfe der elektrischen Signale von dem Herz, die durch eine Vielzahl von Oberflächenelektroden, welche auf einer äußeren Oberfläche des Lebewesens angeordnet sind, erfasst werden, wobei das Computerprogramm Programmcodemittel umfasst, um ein System nach Anspruch 2 zu veranlassen, die Schritte des Verfahrens nach Anspruch 6 durchzuführen, wenn das Computerprogramm auf einem Computer zur Steuerung des Systems ausgeführt wird.

## Revendications

1. Gilet destiné à être porté par un être vivant, le gilet étant adapté pour être utilisé pour fournir une carte d'activité électrique du coeur d'un être vivant (30), le gilet (108) comprenant :
- une pluralité d'électrodes superficielles (109) destinées à être agencées sur une surface extérieure de l'être vivant (30), lorsque le gilet (108) est porté par l'être vivant (30), et pour acquérir des signaux électriques à partir du coeur de l'être vivant (30),
- une fibre de détection de forme optique (107) pour générer un signal de détection de forme optique indicatif de la forme tridimensionnelle de la fibre de détection de forme optique (107) et pour fournir le signal de détection de forme optique à une unité de calcul de positions d'électrodes superficielles.

2. Système pour fournir une carte d'activité électrique du coeur d'un être vivant au moyen de signaux électriques à partir du coeur acquis par une pluralité d'électrodes superficielles agencées sur une surface extérieure de l'être vivant, dans lequel le système est adapté pour être utilisé avec un gilet selon la revendication 1 destiné à être porté par l'être vivant (30) et comprenant la pluralité d'électrodes superficielles, le système comprenant :
- une unité de fourniture de relation spatiale (112) pour fournir des relations spatiales entre la fibre de détection de forme optique (107) du gilet (108) et les électrodes superficielles (109),
- une unité de calcul de positions d'électrodes superficielles (113) pour calculer les positions des électrodes superficielles (109) en fonction de la forme tridimensionnelle de la fibre de détection de forme optique (107) telle qu'elle est indiquée par le signal de détection de forme optique fournie par la fibre de détection de forme optique (107) et de la relation spatiale fournie,
- une unité de calcul de position de structure cardiaque (114) pour calculer la position d'une structure cardiaque d'un être vivant en fonction a) du signal de détection de forme optique reçu à partir de la fibre de détection de forme optique (107) encastrée dans le gilet, b) d'un signal échographique reçu à partir d'une unité échographique (122) équipée d'une fibre de détection de forme optique (110), dans lequel le signal échographique est indicatif de la position de la structure cardiaque, et c) du signal de détection de forme optique reçu à partir de la fibre de détection de forme optique (110) de l'unité échographique (122), dans lequel le signal de détection de forme optique est indicatif de la position de l'unité échographique (122),
- une unité de détermination de carte d'activité électrique (116) pour déterminer la carte d'activité électrique au niveau de la structure cardiaque en fonction des signaux électriques mesurés sur la surface extérieure de l'être vivant (30), des positions déterminées de la pluralité d'électrodes (109) et de la position déterminée de la structure cardiaque.

3. Système selon la revendication 2, dans lequel le système comprend l'unité échographique (122) pour générer le signal échographique indicatif de la position de la structure cardiaque, dans lequel l'unité échographique (122) est équipée du capteur de détection de forme optique (110) pour générer le signal de détection de forme optique indicatif de la position de l'unité échographique (122).

4. Système selon la revendication 3, dans lequel l'unité échographique (122) est une sonde échographique transthoracique ou une sonde échographique transoesophagienne.

5. Système selon la revendication 3, dans lequel le signal échographique représente une image échographique et dans lequel l'unité de calcul de structure cardiaque (14) est adaptée pour détecter la structure cardiaque dans l'image échographique et pour calculer la position de la structure cardiaque en fonction de la structure cardiaque détectée dans l'image échographique.

6. Procédé pour fournir une carte d'activité électrique du coeur d'un être vivant au moyen de signaux électriques à partir du coeur acquis par une pluralité d'électrodes superficielles agencées sur une surface extérieure de l'être vivant, le procédé comprenant :
- la détermination de positions de la pluralité d'électrodes superficielles, dans lequel un gilet (108) selon la revendication 1 est porté par l'être vivant (30) et comprend la pluralité d'électrodes superficielles (109), dans lequel des relations spatiales entre la fibre de détection de forme optique (107) du gilet (108) et les électrodes superficielles (109) sont fournies par une unité de fourniture de relation spatiale (112), et dans lequel les positions des électrodes superficielles (109) sont calculées par une unité de calcul de positions d'électrodes superficielles (113) en fonction d'une forme tridimensionnelle de la fibre de détection de forme optique (107) telle qu'elle est indiquée par le signal de détection de forme optique fourni par la fibre de détection de forme optique (107) et de la relation spatiale fournie,
- la détermination d'une position d'une structure cardiaque de l'être vivant par une unité de calcul de position de structure cardiaque (114), dans lequel la position de la structure cardiaque est déterminée en fonction a) du signal de détection de forme optique reçu à partir de la fibre de détection de forme optique (107) encastrée dans le gilet, b) d'un signal échographique reçu à partir d'une unité échographique transthoracique (122) équipée d'une fibre de détection de forme optique (110), dans lequel le signal échographique est indicatif de la position de la structure cardiaque, et c) du signal de détection de forme optique reçu à partir du capteur de détection de forme optique (110) de l'unité échographique (122), dans lequel le signal de détection de forme optique est indicatif de la position de l'unité échographique (122),
- la détermination de la carte d'activité électrique au niveau de la structure cardiaque par une unité de détermination de carte d'activité électrique en fonction des signaux électriques mesurés sur la surface extérieure de l'être vivant, des positions déterminées de la pluralité d'électrodes et de la position déterminée de la structure cardiaque.

7. Programme d'ordinateur pour fournir une carte d'activité électrique du coeur d'un être vivant au moyen de signaux électriques à partir du coeur acquis par une pluralité d'électrodes superficielles agencées sur une surface extérieure de l'être vivant, le programme d'ordinateur comprenant des moyens codes de programme pour faire en sorte qu'un système selon la revendication 2 réalise les étapes du procédé selon la revendication 6, lorsque le programme d'ordinateur est exécuté sur un ordinateur commandant le système.
